# EUROPEAN PATENT SPECIFICATION

(11) **EP 1 107 725 B1**
(45) Date of publication and mention of the grant of the patent: **19.05.2004**
(21) Application number: 99938351.6
(22) Date of filing: 23.07.1999
(51) Int. Cl.: A61K 7/16

(54) **TOOTHPASTE COMPRISING FINE AND COARSE CALCIUM CARBONATE**
ZAHNPASTA AUF BASIS VON FEINTEILIGEM UND GROSSTEILIGEM KALZIUMCARBONAT
PATE DENTIFRICE CONTENANT DU CARBONATE DE CALCIUM A PARTICULES FINES ET GROSSIERES

(30) Priority: 24.08.1998 EP 98306759
(43) Date of publication of application: 20.06.2001
(73) Proprietor: UNILEVER N.V., 3013 AL Rotterdam (NL); UNILEVER PLC, London EC4P 4BQ (GB)
(72) Inventor: ASHCROFT, Alexander, Thomas, Mollingros, Merseyside CH1 6CD (GB); GIBBS, Christopher, David, Bebington, Merseyside L63 3JW (GB); WATERFIELD, Philip, Christopher, Bebington, Merseyside L63 3JW (GB); SINGLETON, Stephen, John, Moreton, irral CH46 0RF (GB)
(74) Representative: Rosen Jacobson, Frans Lucas M.
(86) International application number: PCT/EP1999/005362
(87) International publication number: WO 2000/010520

(56) References cited:
- EP-A- 0 219 483
- WO-A-96/09034
- FR-A- 2 063 011
- FR-A- 2 625 676
- GB-A- 1 449 317
- US-A- 4 828 820
- CHEMICAL ABSTRACTS, vol. 78, no. 2, 15 January 1973 (1973-01-15) Columbus, Ohio, US; abstract no. 7807n, Y. AOYAMA: "Effect of dentifrices on abrasion of the tooth. III. Comparison of the abrasive activity of various powders" page 240; XP002097409 & KITANO BYOIN KIYO, vol. 17, no. 1-2, 1972, pages 40-46,

## Description

The present invention relates to a toothpaste which contains, in a suitable liquid or pasty medium, particulate calcium carbonate as the main abrasive cleaning agent.

Particulate calcium carbonate is a well-know abrasive cleaning agent in toothpastes, and toothpastes with this abrasive cleaning agent have been on the market for decades. Various types and sources of particulate calcium carbonate have been used or suggested, such as particulate natural calcium carbonates, examples of which are powdered limestone, milled marble, ground dolomite, ground vaterite, ground aragonite and prepared chalk, as well as synthetic particulate calcium carbonates such as precipitated calcium carbonates. The synthetic particulate calcium carbonates are more commonly used and are usually preferred over particulate natural calcium carbonates. The average particle size of the various particulate calcium carbonates used can vary over a wide range, and usually the weight median diameter is 40 microns or less.

One of the drawbacks of particulate calcium carbonates as abrasive cleaning agents is, that although they have a good cleaning action on the teeth they can be rather abrasive and damage the enamel and the dentine of the teeth more than other abrasive cleaning agents such as dicalciumphosphate and insoluble sodium metaphosphate.
It is also known that this abrasiveness of particulate calcium carbonates increases with increasing weight average particle sizes, reason why usually low weight average particle sizes in the range of 1-15 microns are used.

It has now surprisingly and unexpectedly been found that the inclusion of a small amount of much coarser particulate calcium carbonates in the particulate calcium carbonates with a weight average particle size of 15 microns or less reduces the abrasiveness of the latter while maintaining the same cleaning efficacy. Thus, it has been found that a mixture of 75-92.5% by weight of particulate calcium carbonates with a weight average particle size of 15 microns or less (hereinafter referred to as "fine calcium carbonates"), and 25-7.5% by weight of particulate calcium carbonates with a weight average particle size of 30 microns or more (hereinafter referred to as "coarse calcium carbonates"), produces the same cleaning efficacy, but a reduced abrasiveness in comparison with 100 % of the particulate calcium carbonates with a weight average particle size of 15 microns or less.

The coarse calcium carbonates have a weight average particle size in the range of 30 microns to 120 microns, preferably 50 to 100 microns and particularly preferably 70 to 90 microns. The level of the coarse calcium carbonates in the total particulate calcium carbonate mixture is from 7.5-25 % by weight, preferably from 10-20 % by weight and particularly preferably from 12.5-17.5 % by weight of the mixture.

The fine calcium carbonates have a weight average particle size of between 1 and 15 microns, preferably 2-10 microns. The total amount of fine and coarse calcium carbonates, used as abrasive cleaning agent in the toothpaste of the invention, ranges from 25 to 60 % by weight of the toothpaste, preferably 35 to 50 % by weight.

The coarse and the fine particulate calcium carbonates can each be of either natural or synthetic origin. The mixture of the coarse and fine particulate calcium carbonates can be obtained by simply mixing the proper amounts of the respective materials; when they are of the same origin it is also possible to classify a source of particulate calcium carbonates into two fraction having the required weight average particle size, and subsequently mixing the two fractions in the required proportions. The fine and coarse materials can, of course, also be added separately to the toothpaste during the latter's manufacture.

The particle sizes are measured using a Malvern Mastersizer Model X version 1.2a, using the measurement procedure outlined in the instruction manual, using a 300 mm lens in the detector system. Where in this specification reference is made to the D₅₀, this means the particle size, 50 % by weight of the total amount of particles is bigger than and 50 % by weight of the total amount of particles is smaller than.

The toothpaste of the present invention comprises a liquid or pasty medium which may furthermore comprise optional, conventional ingredients such as pharmaceutically acceptable carriers like starch, sucrose, water or water/alcohol systems etc.. Small amounts of surfactants may also be included, such as anionic, nonionic, cationic and amphoteric surfactants. When formulated into a dentifrice, such formulation may contain all the usual dentifrice ingredients.

Thus, it may comprise additional particulate abrasive materials such as silicas, aluminas, dicalciumphosphates, calcium pyrophosphates, hydroxyapatites, trimetaphosphates, insoluble hexametaphosphates and so on, including agglomerated particulate abrasive materials, in amounts less than the amount of calcium carbonate, the total amount of abrasive material not being more than 60% by weight of the composition.

Furthermore, the toothpaste may comprise humectants such as glycerol, sorbitol, propyleneglycol, xylitol, lactitol and so on.

Binders and thickeners such as sodium carboxymethylcellulose, xanthan gum, gum arabic, thickening silicas etc. may also be included.

Flavours such as peppermint and spearmint oils may also be included, as well as preservatives, opacifying agents, colouring agents, pH-adjusting agents, sweetening agents, teeth whitening agents, peroxy bleaching agents and so on. Stabilising agents for peroxy bleaching agents such as dipicolinic acid or sodium stannate may also be usefully included. Furthermore, fully neutralized polyacrylates may be included to improve the compatibility of the calcium carbonates with fluorine compounds.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole.
Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate).

Furthermore anti-inflammatory agents such as ibuprofen, flurbiprofen, aspirin, indomethacin etc. may also be included.

Anti-caries agents such as sodium fluoride, sodium monofluorophosphate, stannous fluoride, aminefluorides, plaque buffers such as urea, calcium lactate, calcium glycerophosphate, strontium polyacrylates may also be included. Other optional ingredients include vitamins such as Vitamin C, and plant extracts. Desensitising agents such as glycerolmonooleate, potassium citrate, potassium chloride, potassium tartrate, potassium bicarbonate, potassium oxalate, potassium nitrate as well as strontium salts may also be included.

Buffers and salts to buffer the pH and ionic strength of the toothpaste may also be included such as mono- or trisodium orthophosphate. Liposomes and other encapsulates may also be used to improve delivery or stability of active ingredients.

Furthermore, the toothpaste may comprise anti-calculus agents such as alkalimetal pyrophosphates, hypophosphite-containing polymers, organic phosphonates, phosphocitrates etc..

In addition, the toothpaste may comprise functional biomolecules such as bacteriocins, antibodies, enzymes and so on.

Other optional ingredients that may be included are e.g. bleaching agents, e.g. those described in EP-A-0,545,594, effervescing systems such as sodium bicarbonate/citric acid systems, colour change systems, and so on.

The toothpaste of the present invention may be formulated into a single formulation, or it may be formulated for multi compartment containers into different formulations, e.g. one containing the calcium carbonate and ingredients compatible therewith, and another containing the remaining ingredients.

The present invention will further be illustrated by way of Example.

### Example 1

The Relative Dentine Abrasion (RDA) values of the following toothpastes were measured by the RDA-method of Grabenstetter, et al., described in J. Dent. Res. 37 (1958), pages 1060-1068.

The cleaning potential of these pastes was measured by the in-vitro stain model method as described by Stookey in J. Dent. Res. 61(11) (1982), pages 1236-1239.

The toothpastes had the following compositions:

| Composition | A | B | C |
|---|---|---|---|
| Particulate chalk (D₅₀ = 8 micron) | 35 | 32.5 | 30 |
| Particulate chalk (D₅₀ = 76 micron) | - | 2.5 | 5 |
| Trisodium orthophosphate | 1 | 1 | 1 |
| Thickening silica | 2 | 2 | 2 |
| Sorbitol (70 %) | 30 | 30 | 30 |
| Sodium carboxymethylcellulose | 0.9 | 0.9 | 0.9 |
| Sodium laurylsulphate | 2.5 | 2.5 | 2.5 |
| Titanium dioxide | 0.5 | 0.5 | 0.5 |
| Formalin | 0.075 | 0.075 | 0.075 |
| Sodium saccharin | 0.22 | 0.22 | 0.22 |
| Flavour | 1.1 | 1.1 | 1.1 |
| Water | q.s. | q.s. | q.s. |

The following results were obtained:

| | A | B | C |
|---|---|---|---|
| RDA-value | 113 | 122 | 86 |
| | | | |
| Between A and B, no significant difference; | | | |
| between A and C a significant difference (P<0.05) | | | |
| | | | |
| % cleaning (after 750 strokes) | 55 | 65 | 50 |
| % cleaning (after 1500 strokes) | 55 | 70 | 55 |
| No significant differences between A, B and C. | | | |

These results show a significantly reduced RDA for product C, and the maintenance of the cleaning efficacy of the toothpaste of the invention.

## Claims

1. A toothpaste comprising, in a liquid or pasty medium, particulate calcium carbonate as the main abrasive cleaning agent, **characterized in that** the particulate calcium carbonate comprises a mixture of 75-92.5 % by weight of the mixture fine of particulate calcium carbonate with a weight average particle size of between 1 and 15 microns, and 7.5-25 % by weight of the mixture of coarse particulate calcium carbonate with a weight average particle size of between 30 and 120 microns.

2. A toothpaste according to claim 1, **characterized in that** the fine particulate calcium carbonate has a weight average particle size of between 2 and 10 microns.

3. A toothpaste according to claim 1 or 2, **characterized in that** the coarse particulate calcium carbonate has a weight average particle size of between 50 and 100 microns.

4. A toothpaste according to claims 1-3, **characterized in that** the coarse particulate calcium carbonate has a weight average particle size of between 70 and 90 microns.

5. A toothpaste according to claim 1-4, **characterized in that** it contains from 10-20 % by weight of the mixture of coarse particulate calcium carbonate.

6. A toothpaste according to claim 5, **characterized in that** it contains 12.5-17.5 % by weight of the mixture of coarse particulate calcium carbonate.

7. A toothpaste according to claims 1-6, **characterized in that** the toothpaste comprises from 25 to 60 % by weight of the toothpaste of the particulate calcium carbonate mixture.

8. A toothpaste according to claim 7, **characterized in that** it contains from 35 to 50 % by weight of the toothpaste of the particulate calcium carbonate mixture.

## Patentansprüche

1. Zahnpasta enthaltend in einem flüssigen oder pastenförmigen Medium teilchenförmiges Calciumcarbonat als hauptsächliches abrasives Reinigungsmittel, **dadurch gekennzeichnet, dass** das teilchenförmige Calciumcarbonat eine Mischung aufweist mit 75 bis 92,5 Gew.-% der Mischung an feinem teilchenförmigen Calciumcarbonat mit einer gewichtsmittleren Teilchengröße zwischen 1 und 15 µm und 7,5 bis 25 Gew.-% der Mischung an grobem teilchenförmigen Calciumcarbonat mit einer gewichtsmittleren Teilchengröße zwischen 30 und 120 µm.

2. Zahnpasta nach Anspruch 1, **dadurch gekennzeichnet, dass** das feine teilchenförmige Calciumcarbonat eine gewichtsmittlere Teilchengröße von 2 bis 10 µm hat.

3. Zahnpasta nach Anspruch 1 oder Anspruch 2, **dadurch gekennzeichnet, dass** das grobe teilchenförmige Calciumcarbonat eine gewichtsmittlere Teilchengröße von 50 bis 100 µm hat.

4. Zahnpasta nach einem der Ansprüche 1 bis 3, **dadurch gekennzeichnet, dass** das grobe teilchenförmige Calciumcarbonat eine gewichtsmittlere Teilchengröße von 70 bis 90 µm hat.

5. Zahnpasta nach einem der Ansprüche 1 bis 4, **dadurch gekennzeichnet, dass** sie 10 bis 20 Gew.-% der Mischung an grobem teilchenförmigen Calciumcarbonat enthält.

6. Zahnpasta nach Anspruch 5, **dadurch gekennzeichnet, dass** sie 12,5 bis 17,5 Gew.-% der Mischung an grobem teilchenförmigen Calciumcarbonat enthält.

7. Zahnpasta nach einem der Ansprüche 1 bis 6, **dadurch gekennzeichnet, dass** die Zahnpasta 25 bis 60 Gew.-% der Zahnpasta an teilchenförmiger Calciumcarbonatmischung enthält.

8. Zahnpasta nach Anspruch 7, **dadurch gekennzeichnet, dass** sie 35 bis 50 Gew.-% der Zahnpasta an teilchenförmiger Calciumcarbonatmischung enthält.

## Revendications

1. Pâte dentifrice contenant un milieu liquide ou pâteux, du carbonate de calcium particulaire en tant qu'agent nettoyant abrasif principal, **caractérisée en ce que** le carbonate de calcium particulaire comprend un mélange de 75 - 92,5 % en poids, sur la base du mélange, de carbonate de calcium particulaire fin avec une taille de particule moyenne pondérée comprise entre 1 et 15 microns, et de 7,5 à 25 % en poids, sur la base du mélange, de carbonate de calcium particulaire grossier, avec une taille de particule moyenne pondérée comprise entre 30 et 120 microns.

2. Pâte dentifrice selon la revendication 1, **caractérisée en ce que** le carbonate de calcium particulaire fin a une taille de particule moyenne pondérée comprise entre 2 et 10 microns.

3. Pâte dentifrice selon la revendication 1 ou 2, **caractérisée en ce que** le carbonate de calcium particulaire grossier a une taille de particule moyenne pondérée comprise entre 50 et 100 microns.

4. Pâte dentifrice selon les revendications 1 à 3, **caractérisée en ce que** le carbonate de calcium particulaire grossier a une taille de particule moyenne pondérée comprise entre 70 et 90 microns.

5. Pâte dentifrice selon les revendications 1 à 4, **caractérisée en ce qu'**elle contient de 10 à 20 % en poids du mélange de carbonate de calcium particulaire grossier.

6. Pâte dentifrice selon la revendication 5, **caractérisée en ce qu'**elle contient de 12,5 à 17,5 % en poids du mélange de carbonate de calcium particulaire grossier.

7. Pâte dentifrice selon les revendications 1 à 6, **caractérisée en ce que** la pâte dentifrice comprend de 25 à 60 % en poids, sur la base de la pâte dentifrice, du mélange de carbonate de calcium particulaire.

8. Pâte dentifrice selon la revendication 7, **caractérisée en ce qu'**elle contient de 35 à 50 % en poids, sur la base de la pâte dentifrice, du mélange de carbonate de calcium particulaire.
